# EUROPEAN PATENT SPECIFICATION

(11) **EP 0 238 211 B1**
(45) Date of publication and mention of the grant of the patent: **12.01.1994**
(21) Application number: 87301434.4
(22) Date of filing: 19.02.1987
(51) Int. Cl.: C07D 211/90, A61K 31/44

(54) **Dihydropyridines, process for their preparation and their use as medicaments**
Dihydropyridine, Verfahren zu ihrer Herstellung und ihre Verwendung als Arzneimittel
Dihydropyridines, leur procédé de préparation et leur utilisation comme médicaments

(30) Priority: 20.02.1986 IT 1948586
(43) Date of publication of application: 23.09.1987
(73) Proprietor: GLAXO S.p.A., Verona (IT)
(72) Inventor: Semeraro, Claudio, Bresso (Milano) (IT); Micheli, Dino, Verona (IT); Pieraccioli, Daniele, Verona (IT); Gaviraghi, Giovanni, Verona (IT); Borthwick, Alan David, London NW11 7UA (GB)
(74) Representative: Marchant, James Ian

(56) References cited:
- EP-A- 071 819
- EP-A- 0 012 180
- EP-A- 0 063 365
- DE-A- 3 207 982
- DE-A- 3 529 997
- US-A- 3 455 945
- DEUTSCHE APOTHEKER ZEITUNG 104, 1983, pages 139-146; R. MANNHOLD et al.: "Pharmakologische Differenzierung von Calcium-Antagonisten"
- CHEMICAL & PHARMACEUTICAL BULLETIN, vol. 27, no. 5, 1979, pages 1426-1440; T. KAMEYAMA et al.: "Effect of centrally Acting Muscle Relaxants on the Morphine-Induced Straub Tail Reaction in Mice"
- Aug. Chem. Int. Ed. 20, 762(1981)
- J. Med. Chem. 26, 775(1983)
- Drugs of the Future VI, 427(1981)

## Description

This invention relates to novel heterocyclic derivatives which have an effect on the transmembranal influx of calcium ions into the cells of cardiac and smooth muscle, to processes for the preparation thereof, to pharmaceutical compositions containing them and to their use in therapeutics.

The role of intracellular calcium ions in the control of the contractile system of cardiac and smooth muscle is well known. It has been established that compounds which limit the intracellular calcium ion concentration by preventing or reducing the transmembranal calcium ion influx in cells of the contractile system of cardiac and smooth muscle are useful in the treatment of cardiovascular disorders.

We have now found a new group of compounds which reduce intracellular calcium ion concentration by limiting transmembranal calcium ion influx and thus may be useful for the treatment of cardiovascular disorders such as hypertension, angina pectoris, myocardial ischaemia, congestive heart failure, cerebral vascular and peripheral disorders, and for the treatment of diseases characterised by reversible airway obstruction such as asthma and chronic bronchitis.

The present invention provides 1,4-dihydropyridine derivatives with a phenyl group at the 4-position having a -CH=CR₆CO₂R₅ substituent at the ortho position to the point of attachment to the dihydropyridine ring (R₅ and R₆ being as defined below).

US-A-3,455,945 discloses 1,4-dihydropyridine derivatives in which the substituent at the 4-position is a phenyl group substituted by a carboxyl or carboalkoxy group. There is no teaching that the carboalkoxy group attached directly to the phenyl ring could be modified to provide the 4-position substituent of the compounds of the present invention.

DE-A-3,207,982 teaches a wide range of 1,4-dihydropyridines in which the substituent at the 4-position may be inter alia a benzene ring. The said benzene ring carries the group XBY. The group X may represent a direct bond, oxygen, sulphur or a sulphoxide group, B may be a bond or an alkylene group and Y may represent a large number of groups including an alkenyl group containing at least six carbon atoms and the said Y group may also be substituted by one to three groups selected from a range of substituents including carboalkoxy. There is no teaching of any alkenyl group substituted by any other group, let alone by carboalkoxy.

EP-A-0,071,819 describes a very wide range of 1,4-dihydropyridine derivatives. The substituent at the 4-position may represent an aryl radical substituted by an alkenyl group or a group COOR^{v} wherein R^{v} may be an alkyl group, or the substituent at the 4-position may represent an aryl radical substituted by an alkyl group which in turn may be substituted by the group COOR^{v}. There is no suggestion to extend this very wide definition even further to include 4-substituents in which the phenyl group is substituted by an alkenyl group carrying a carboalkoxy group.

The invention thus provides for compounds of the general formula (I) 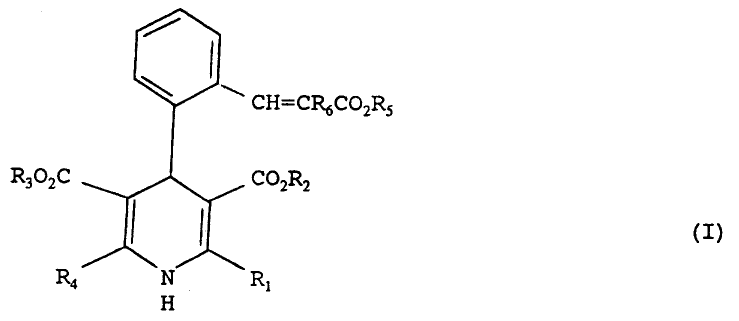
and physiologically acceptable salts thereof,
in which
R₁ and R₄ independently represent a C₁₋₄ alkyl group;
R₂ represents a group 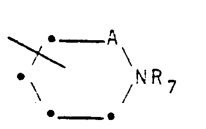
(where A is a bond or a methylene group and R₇ is phenyl C₁₋₄ alkyl); or R₂ represents a group CH₂CH₂NR₈R₉ (where R₈ is hydrogen or C₁₋₄ alkyl and R₉ is C₁₋₄ alkyl, phenylC₁₋₄alkyl or benzoylC₁₋₄alkyl); or R₂ represents a C₁₋₄ alkyl group substituted by nitrile;
R₃ represents a C₁₋₆ straight or branched chain alkyl or alkoxyalkyl group;
R₅ represents a C₂₋₉ straight or branched chain alkyl group;
and
R₆ represents a hydrogen or halogen atom or a C₁₋₃ alkyl group.

The compounds represented by formula (I) can exist in more than one isomeric and/or enantiomeric form and the invention includes all such isomers, enantiomers and mixtures thereof.

The term 'alkyl' as a group or part of a group means that the group is straight or branched.

The compounds of formula (I) in which the group R₂ is basic forms salts with inorganic or organic acids. Particularly suitable salts are those of physiologically acceptable inorganic and organic acids and include hydrochlorides, hydrobromides, sulphates, p-toluenesulphonates, methanesulphonates, formates, acetates, maleates, fumarates, succinates, phosphates, citrates, tartrates and benzoates.

Examples of suitable groups for R₁ and R₄ independently include methyl and ethyl groups.

Examples of suitable groups for R₂ include N-benzylpyrrolidino, N-benzylpiperidino, CH₂CH₂CN and CH₂CH₂NR₈R₉ (where R₈ is hydrogen or methyl and R₉ is methyl, benzyl or benzoylethyl).

Examples of suitable groups for R₃ include C₁₋₄ straight or branched chain alkyl groups such as methyl, ethyl, isopropyl, isobutyl, t-butyl or C₁₋₄ alkyl (such as ethyl) substituted by a C₁₋₃ alkoxy (e.g. methoxy or propoxy) group.

The group R₅ may for example be a methyl, ethyl, propyl, isopropyl, butyl, sec butyl, isobutyl, tert butyl, pentyl, isopentyl, neopentyl, hexyl, 2,6-dimethyl-4-heptyl or octyl group.

When the group R₆ represents a C₁₋₃ alkyl group this may for example be a methyl, ethyl or n-propyl, and is preferably a methyl or ethyl group.

When the group R₆ represents a halogen atom this may be for example a chlorine, bromine or iodine atom, and is preferably a bromine atom.

The group -CH=CR₆CO₂R₅ in the compounds of formula (I) can exist in the (Z) or the (E) configuration and preferred compounds are those in which the hydrogen atom and the group R₆ are trans with respect to each other.

Preferably R₁ and R₄ represent methyl groups.

R₂ preferably represents N-benzylpiperidino, CH₂CH₂CN or CH₂CH₂NR₈R₉ (where R₈ is hydrogen or methyl and R₉ is methyl or benzyl).

R₃ preferably represents C₁₋₄ alkyl e.g. methyl or ethyl.

R₅ preferably represents tert butyl.

R₆ preferably represents a methyl or ethyl group or more perfectly a hydrogen atom.

Particularly preferred compounds according to the invention are
2,6-Dimethyl-4-[2-(3-(1,1-dimethylethoxy)-3-oxo-1-propenyl)phenyl]-1,4-dihydro-3,5-pyridine-dicarboxylic acid, ethyl (1-benzyl-4-piperidinyl)ester;
2,6-Dimethyl-4-[2-(3-(1,1-dimethylethoxy)-3-oxo-1-propenyl)phenyl]-1,4-dihydro-3,5-pyridinedicarboxylic acid, ethyl, dimethylaminoethyl ester;
2,6-Dimethyl-4-[2-(3-(1,1-dimethylethoxy)-3-oxo-1-propenyl)phenyl]-1,4-dihydro-3,5-pyridinedicarboxylic acid, ethyl 2-cyano ethylester;
2,6-Dimethyl-4-[2-(3-(1,1-dimethylethoxy)-3-oxo-1-propenyl)phenyl]-1,4-dihydro-3,5-pyridinedicarboxylic acid, methyl (N-phenylmethyl-N-methylaminoethyl) ester;
and more particularly the E isomers thereof, and their physiologically acceptable salts.

The ability of the compounds to limit or inhibit the effect of calcium ions on the tone of vascular smooth muscle maybe determined using a depolarised rabbit ear artery prepared according to the method of Towart. R. et al Br. J. Pharmacol. 1982, 75, 1508.

The antihypertensive activity of the compounds of the invention was demonstrated by intravenous and/or oral administration of the compound to male spontaneously hypertensive rats.

The compounds of the invention are thus of interest in the treatment of hypertension and diseases characterised by reversible airways obstruction such as asthma and chronic bronchitis. They are also potentially useful for the treatment of other cardiovascular disorders including angina pectoris, myocardial ischaemia, congestive heart failure, cerebral vascular and peripheral disorders.

The compounds of the invention may be formulated in a conventional manner for use with one or more pharmaceutical carriers or excipients.

Thus a further aspect of the invention includes pharmaceutical compositions the compounds of formula (I) formulated for oral, sub lingual, transdermal, parenteral or rectal administration or for administration by inhalation or insufflation.

A proposed daily dosage of active compound of the invention for the treatment of man is in the range of 0.03mg to 100mg, which may conveniently be administered in one or more doses. The precise dose employed will depend on the age and condition of the patient as well as the route of administration.

For oral use the compounds of the invention are conveniently administered to the human patient at a dose in the range 0.3 to 100mg per day. For parenteral use the compounds of the invention are conveniently administered at a dose in the range of 0.03-30mg per day.

For administration by inhalation use the compounds of the invention are conveniently administered to the human patient at a dose in the range of 0.1mg to 10mg per day.

For oral use the compound is preferably administered twice or more particularly once a day.

Methods for preparing the compounds of formula (I) are described below and for the intermediates described below Rₗ, R₂, R₃, R₄, R₅ and R₆ have the meanings defined above for compounds of formula (I) or are such groupings in a protected form unless otherwise stated.

Thus compounds of formula (I) may be prepared by reaction the α,β-unsaturated ketone (II) with the aminoester (III) . The reaction is conveniently carried out in a solvent such as an alkanol, e.g. ethanol or isopropanol and preferably with heating e.g. 40-150°C. 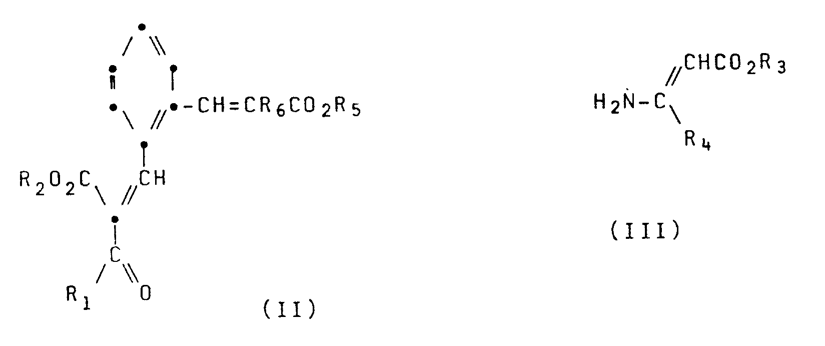
The α,β-unsaturated ketone (II) may be prepared by reacting the aldehyde (IV) with the ketoester (V), in a solvent such as an alkanol e.g. ethanol or isopropanol, preferably with heating e.g. 40-150°C. Conveniently this reaction is carried out in the presence of a catalyst such as piperidine acetate. 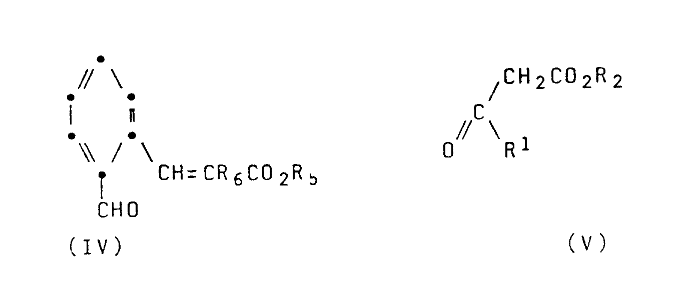
In a modification of this process for preparing compounds of formula (I), the aldehyde (IV) may be reacted with a mixture of the aminoester (III) and the ketoester (V) under the conditions previously described for the reaction of the α,β-unsaturated ketone (II) with the aminoester (III).

Compounds of formula (IV) may be prepared by reacting the bis aldehyde (VI) with the triphenylphosphorane (VII) in solvent such as methylene chloride or toluene. 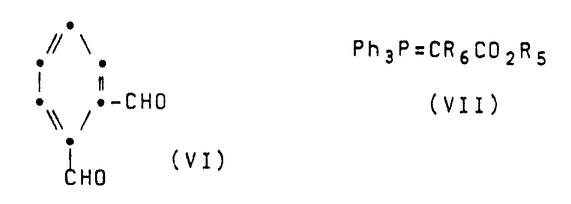
Compounds of formula (IV) may also be prepared by reacting a 2-halobenzaldehyde (VIII) 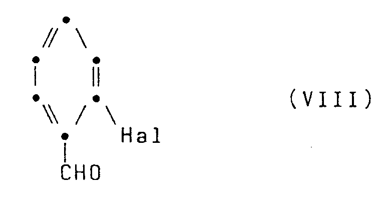
(where Hal represents a bromine or iodine atom) with an acrylic ester CH₂=CR₆CO₂R₅ (IX), in the presence of a catalytic amount of a palladium salt such as palladium acetate, in the presence of a suitable organic base such as a trialkylamine e.g. triethylamine or tri-n-butylamine. The reaction is also preferably carried out in the presence of a triarylphosphine such as tri-otolyphosphine, or more preferably, triphenylphosphine.

The reaction is conveniently carried out in a suitable solvent such as xylene or t-butyl acetate, or more conveniently in dimethylformamide or in a mixture of solvents e.g. xylene/dimethylformamide, preferably with heating. The reaction mixture is preferably heated within the temperature range of 80°C to 150°C, more preferably at 100°C to 110°C.

The compounds of formulae (III), (V), (VI), (VII), (VIII) and (IX) are either known compounds or may be made by analogous processes to those used for known compounds.

The following examples illustrate the invention. Temperatures are in °C.

### Intermediate l

### (E)-3-(2-Formylphenyl)-2-propenoic acid, l,l-dimethyl ethyl ester

A solution of triphenylphosphoranylidene acetic acid l,l-dimethylethyl ester (54.7g) in dry dichloromethane (100ml) was added to a solution of ortho phthaldehyde (19.3g) in dry dichloromethane at 0°C in 15 minutes. The solvent was evaporated and the oil taken up with diethyl ether. The solid triphenylphosphine oxide was filtered, washed with ether and the filtrate evaporated to dryness to give a yellow oil (36g) which was eluted on a silica gel column (petrol ether/diethyl ether, 7:3), to give the title compound as a colourless oil (21.4g).
T.l.c. (Petrol ether/diethyl ether, l:l) Rf 0.45.

### Intermediate 2

### 2-(2-(3-(l,l-Dimethylethoxy)-3-oxo-l-propenyl)phenyl)methylene-3-oxo-butanoic acid, ethyl ester

A solution of piperidine (0.07ml) and acetic acid (0.04ml) in isopropanol (0.58ml) was added to a solution of Intermediate l (3g) and ethyl acetoacetate (0.69g) in isopropanol (8.5ml). The mixture was stirred at 60° for lh, then the solvent was evaporated and the residue taken up with ether. The solution was washed with lN HCl, saturated bicarbonate solution and brine and dried over Na₂SO₄. Evaporation of the solvent gave the title compound as an oil (4.07g; mixture of E and Z isomers).
T.l.c. (petrol ether/ethyl acetate 4:1) Rf 0.26; Rf 0.35.

### Example l

### 2,6-Dimethyl-4(E)-[2-(3-(l,l-dimethylethoxy)-3-oxo-l-propenyl)phenyl]-l,4-dihydro-3,5-pyridinedicarboxylic acid, ethyl, dimethylaminoethyl ester, hydrochloride

A solution of Intermediate l (9.29g), 3-amino-2-butenoic acid ethyl ester (5.18g) and 3-oxo-butanoic acid, dimethylamino ethyl ester (7g) in ethanol (25ml) was refluxed for 15 h. After evaporation of the solvent the residue was purified by column chromatography on silica gel eluting with methylene chloride/methanol 9:1 to give the free base of the title compound (0.6g). A solution of free base (0.25g) and 0.lN HCl (4.9ml) in acetone (9ml) was stirred for five minutes. After evaporation of the solvent the residue was crystallized from petrol to give the title compound (0.2g) as a yellow solid. M.p. 195-196°. T.l.c. (methylene chloride/methanol 9:1) Rf 0.28
Similarly prepared were Examples 2 and 3:

### Example 2

### 2,6-Dimethyl-4(E)-[2-(3-(l,l-dimethylethoxy)-3-oxo-l-propenyl)phenyl]-l,4-dihydro-3,5-pyridinedicarboxylic acid, methyl, N-phenylmethyl-N-methylaminoethyl ester, hydrochloride (0.6g)

From Intermediate l (2.32g), 3-amino-2-butenoic acid, methyl ester (l.15g) and 3-oxo-butanoic acid (2-N-phenylmethyl-N-methylaminoethyl) ester (2.5g). M.p. = 120-130° dec. T.l.c. (ethyl acetate/CH₂Cl₂ 9:1) Rf 0.5

### Example 3

### 2,6-Dimethyl-4(E)-[2-(3-(l,l-Dimethylethoxy)-3-oxo-l-propenyl)phenyl]-l,4-dihydro-3,5-pyridinedicarboxylic acid, ethyl 2-cyanoethyl ester (2.4g)

From Intermediate l (10.5g), 3-oxo-butanoic acid, ethyl ester (5.85g) and 3-amino-2-butenoic acid (2-cyanoethyl) ester (7.5g). M.p. 168-169°. T.l.c. (cyclohexane/ethyl acetate l:l) Rf 0.29

### Example 4

### 2,6-Dimethyl-4(E)-[2-(3-(l,l-dimethylethoxy)-3-oxo-l-propenyl)phenyl]-l,4-dihydro-3,5-pyridinedicarboxylic acid, ethyl (l-benzyl-4-piperidinyl)ester, hydrochloride

A solution of Intermediate 2 (3g) and 3-amino-2-butenoic acid(l-benzyl-4-piperidinyl) ester (2.4g) in ethanol (40ml) was refluxed for ll h. After evaporation of the solvent the residue was purified by column chromatography on silica gel eluting with ethyl acetate/petrol 6:4 to give the free base of' the title compound (0.73g) . A solution of free base (0.73g) and 0.1N HCl (12.15ml) in acetone (10ml) was stirred for 5 min. After evaporation of the solvent the residue was crystallised from petrol to give the title compound (0.63g) as a yellow solid. M.p. 138-140°. T.l.c. (ethyl acetate/petrol 9:1) Rf 0.44

## Claims (Claims for the following Contracting State(s): BE, CH, DE, FR, GB, LI, LU, NL, SE)

1. Compounds of the general formula (I) 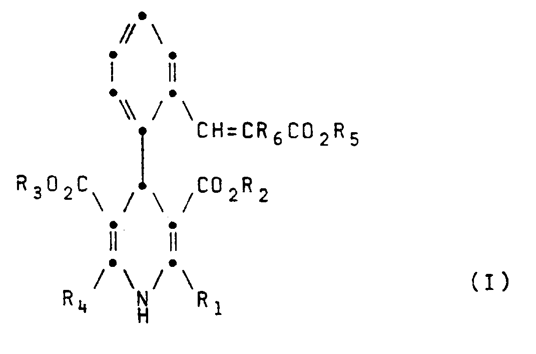 and physiologically acceptable salts thereof, in which
R₁ and R₄ independently represent a C₁₋₄ alkyl group;
R₂ represents a group 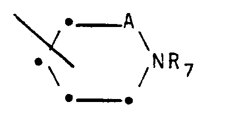 (where A is a bond or a methylene group and R₇ is phenyl C₁₋₄ alkyl); or R₂ represents a group CH₂CH₂NR₈R₉ (where R₈ is hydrogen or C₁₋₄ alkyl and R₉ is C₁₋₄ alkyl, phenyl C₁₋₄ alkyl or benzoyl C₁₋₄ alkyl); or R₂ represents a C₁₋₄ alkyl group substituted by nitrile;
R₃ represents a C₁₋₆ straight or branched chain alkyl or alkoxyalkyl group;
R₅ represents a C₂₋₉ straight or branched chain alkyl group;
and
R₆ represents a hydrogen or halogen atom or a C₁₋₃ alkyl group.

2. Compounds as claimed in Claims 1 in which R₁ and R₄ represent methyl.

3. Compounds as claimed in Claims 1 or 2 in which R₂ represents N-benzylpiperidino, CH₂CH₂CN or CH₂CH₂NR₈R₉ (where R₈ is hydrogen or methyl and R₉ is methyl or benzyl).

4. Compounds as claimed in any of Claims 1 to 3 in which R₃ represents a methyl or ethyl group.

5. Compounds as claimed in any of Claims 1 to 4 in which R₅ represents a tert butyl group.

6. Compounds as claimed in any of Claims 1 to 5 in which R₆ represents a hydrogen atom.

7. A compound selected from
2,6-Dimethyl-4-[2-(3-(1,1-dimethylethoxy)-3-oxo-1-propenyl)phenyl]-1,4-dihydro-3,5-pyridinedicarboxylic acid, ethyl (1-benzyl-4-piperidinyl)ester;
2,6-Dimethyl-4-[2-(3-(1,1-dimethylethoxy)-3-oxo-1-propenyl)phenyl]-1,4-dihydro-3,5-pyridinedicarboxylic acid, ethyl, dimethylaminoethyl ester;
2,6-Dimethyl-4-[2-(3-(1,1-dimethylethoxy)-3-oxo-1-propenyl)phenyl]-1,4-dihydro-3,5-pyridinedicarboxylic acid, ethyl 2-cyanoethylester;
2,6-Dimethyl-4-[2-(3-(1,1-dimethylethoxy)-3-oxo-1-propenyl)phenyl]-1,4-dihydro-3,5-pyridinedicarboxylic acid, methyl-N-phenylmethyl-N-methylaminoethylester;
and their physiologically acceptable salts

8. A compound as claimed in Claims 1 to 7 in which the hydrogen atom and the group R₆ in the moiety -CH=CR₆CO₂R₅ are trans with respect to each other.

9. A process for the preparation of a compound of general formula (I) as defined in any of Claims 1 to 8 which process comprises:
(a) reacting a compound of formula (II) in which R₁, R₂, R₅ and R₆ have the meanings defined in Claim 1 with the amino ester (III) in which R₃ and R₄ have the meanings defined in Claim 1. 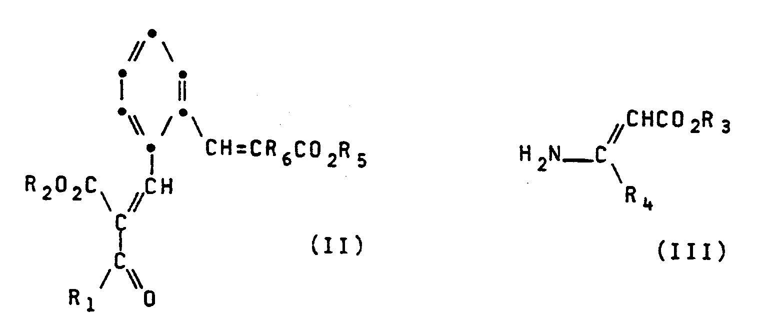
(b) reacting the aldehyde of formula (IV) with an aminoester of formula (III) and the ketoester of formula (V), in which the respective groups R₆, R₅, R₄, R₃, R₂ and R₁ have the meanings defined in Claim 1

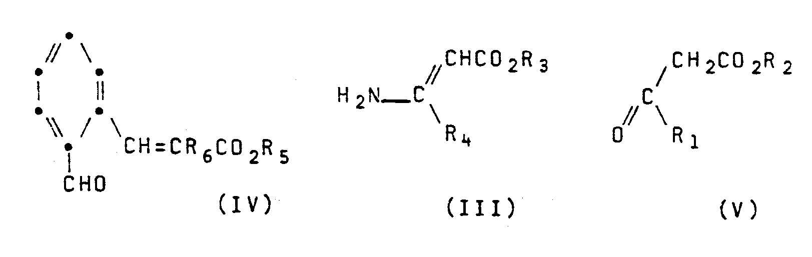

10. Pharmaceutical compositions comprising a compound as claimed in any of Claims 1 to 8 in association with a pharmaceutical acceptable carrier or diluent.

11. Compositions as claimed in Claim 10 in a form suitable for oral, sub lingual, transdermal, parenteral or rectal administration, or for administration by inhalation or insufflation.

12. A compound as claimed in any of Claims 1 to 8 or a physiologically acceptable salt thereof for use as an active therapeutic agent.

## Claims (Claims for the following Contracting State(s): AT, ES, GR)

1. A process for preparing compounds of the general formula (I) 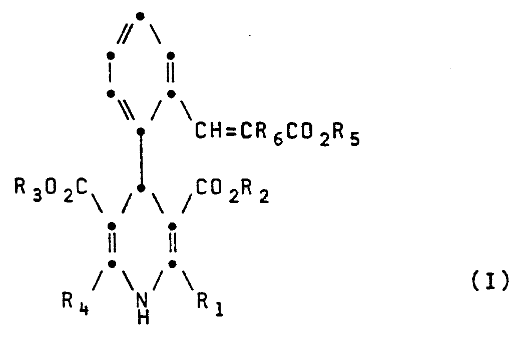 and physiologically acceptable salts thereof, in which
R₁ and R₄ independently represent a C₁₋₄ alkyl group;
R₂ represents a group 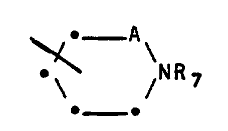 (where A is a bond or a methylene group and R₇ is phenyl C₁₋₄ alkyl); or R₂ represents a group CH₂CH₂NR₈R₉ (where R₈ is hydrogen or C₁₋₄ alkyl and R₉ is C₁₋₄ alkyl, phenyl C₁₋₄ alkyl or benzoyl C₁₋₄ alkyl); or R₂ represents a C₁₋₄ alkyl group substituted by nitrile;
R₃ represents a C₁₋₆ straight or branched chain alkyl or alkoxyalkyl group;
R₅ represents a C₂₋₉ straight or branched chain alkyl group;
and
R₆ represents a hydrogen or halogen atom or a C₁₋₃ alkyl group. which comprises :
(a) reacting a compound of formula (II) in which R₁, R₂, R₅ and R₆ have the meanings defined in formula (I) with the amino ester (III) in which R₃ and R₄ have the meanings defined in formula (I) 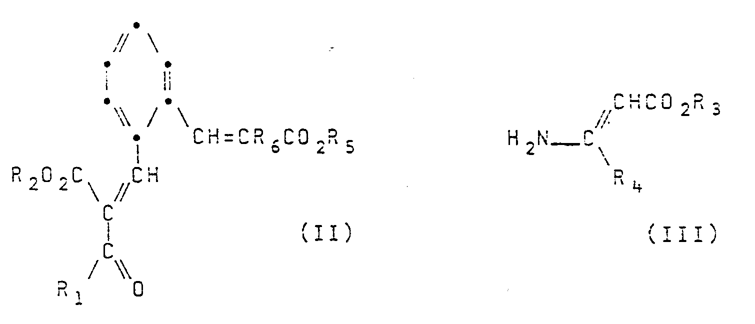
(b) reacting the aldehyde of formula (IV) with an aminoester of formula (III) and the ketoester of formula (V), in which the respective groups R₆, R₅, R₄, R₃, R₂ and R₁ have the meanings defined in formula (I)

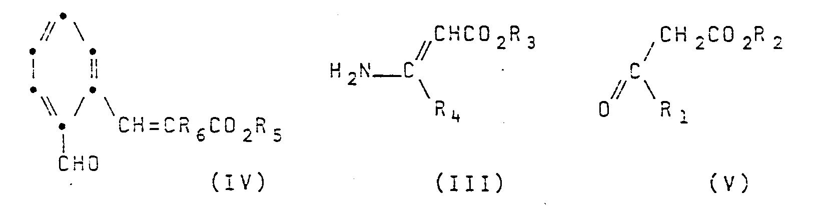

2. A process as claimed in Claims 1 for preparing compounds in which R₁ and R₄ represent methyl.

3. A process as claimed in Claims 1 or 2 for preparing compounds in which R₂ represents N-benzylpiperidino, CH₂CH₂CN or CH₂CH₂NR₈R₉ (where R₈ is hydrogen or methyl and R₉ is methyl or benzyl).

4. A process as claimed in any of Claims 1 to 3 for preparing compounds in which R₃ represents a methyl or ethyl group.

5. A process as claimed in any of Claims 1 to 4 for preparing compounds in which R₅ represents a tert butyl group.

6. A process as claimed in any of Claims 1 to 5 for preparing compounds in which R₆ represents a hydrogen atom.

7. A process as claimed in any of Claims 1 to 6 for preparing a compound selected from
2,6-Dimethyl-4-[2-(3-(1,1-dimethylethoxy)-3-oxo-1-propenyl)phenyl]-1,4-dihydro-3,5-pyridinedicarboxylic acid, ethyl (1-benzyl-4-piperidinyl)ester;
2,6-Dimethyl-4-[2-(3-(1,1-dimethylethoxy)-3-oxo-1-propenyl)phenyl]-1,4-dihydro-3,5-pyridinedicarboxylic acid, ethyl, dimethylaminoethyl ester;
2,6-Dimethyl-4-[2-(3-(1,1-dimethylethoxy)-3-oxo-1-propenyl)phenyl]-1,4-dihydro-3,5-pyridinedicarboxylic acid, ethyl 2-cyanoethylester;
2,6-Dimethyl-4-[2-(3-(1,1-dimethylethoxy)-3-oxo-1-propenyl)phenyl]-1,4-dihydro-3,5-pyridinedicarboxylic acid, methyl-N-phenylmethyl-N-methylaminoethylester;
and their physiologically acceptable salts

8. A process as claimed in Claims 1 to 7 for preparing a compound in which the hydrogen atom and the group R₆ in the moiety -CH=CR₆CO₂R₅ are trans with respect to each other.

## Patentansprüche (Patentansprüche für folgende(n) Vertragsstaat(en): BE, CH, DE, FR, GB, LI, LU, NL, SE)

1. Verbindungen der allgemeinen Formel (I) 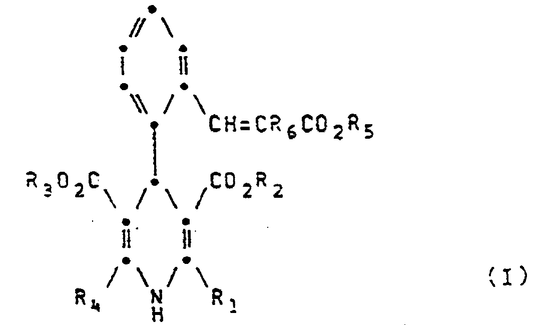 worin R₁ und R₄ unabhängig voneinander für eine C₁₋₄-Alkylgruppe stehen;
R₂ für eine Gruppe 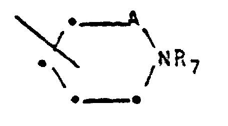 (worin A eine Bindung oder eine Methylengruppe ist und R₇ für Phenyl-C₁₋₄-Alkyl steht) steht; oder R₂ für eine Gruppe CH₂CH₂NR₈R₉ (worin R₈ für Wasserstoff oder C₁₋₄-Alkyl steht und R₉ für C₁₋₄-Alkyl, Phenyl-C₁₋₄-Alkyl oder Benzoyl-C₁₋₄-Alkyl steht) steht; oder R₂ für eine durch Nitril substituierte C₁₋₄-Alkylgruppe steht;
R₃ für eine geradkettige oder verzweigtkettige c₁₋₆-Alkyl- oder Alkoxyalkylgruppe steht;
R₅ für eine geradkettige oder verzweigtkettige c₂₋₉-Alkylgruppe steht; und
R₆ für ein Wasserstoff- oder Halogenatom oder eine C₁₋₃-Alkylgruppe steht,
und die physiologisch annehmbaren Salze davon.

2. Verbindungen nach Anspruch 1, dadurch **gekennzeichnet,** daß R₁ und R₄ für Methyl stehen.

3. Verbindungen nach Anspruch 1 oder 2, dadurch **gekennzeichnet,** daß R₂ für N-Benzylpiperidino, CH₂CH₂CN oder CH₂CH₂NR₈R₉ (worin R₈ für Wasserstoff oder Methyl steht und R₉ für Methyl oder Benzyl steht) steht.

4. Verbindungen nach einem der Ansprüche 1 bis 3, dadurch **gekennzeichnet,** daß R₃ für eine Methyl- oder Ethylgruppe steht.

5. Verbindungen nach einem der Ansprüche 1 bis 4, dadurch **gekennzeichnet,** daß R₅ für eine tert.-Butylgruppe steht.

6. Verbindungen nach einem der Ansprüche 1 bis 5, dadurch **gekennzeichnet,** daß R₆ für ein Wasserstoffatom steht.

7. Verbindung, ausgewählt aus
2,6-Dimethyl-4-[2-(3-(1,1-dimethylethoxy)-3-oxo-1-propenyl)phenyl]-1,4-dihydro-3,5-pyridindicarbonsäureethyl-(1-benzyl-4-piperidinyl)ester;
2,6-Dimethyl-4-[2-(3-(1,1-dimethylethoxy)-3-oxo-1-propenyl)phenyl]-1,4-dihydro-3,5-pyridindicarbonsäureethyldimethylaminoethylester;
2,6-Dimethyl-4-[2-(3-(1,1-dimethylethoxy)-3-oxo-1-propenyl)phenyl]-1,4-dihydro-3,5-pyridindicarbonsäureethyl-2-cyanoethylester;
2,6-Dimethyl-4-[2-(3-(1,1-dimethylethoxy)-3-oxo-1-propenyl)phenyl]-1,4-dihydro-3,5-pyridindicarbonsäuremethyl-N-phenylmethyl-N-methylaminoethylester
und ihren physiologisch annehmbaren Salzen.

8. Verbindung nach den Ansprüchen 1 bis 7, dadurch **gekennzeichnet,** daß das Wasserstoffatom und die Gruppe R₆ in der Gruppierung -CH=CR₆CO₂R₅ bezüglich einander in trans-Konfiguration vorliegen.

9. Verfahren zur Herstellung einer Verbindung der allgemeinen Formel (I) nach eine der Ansprüche 1 bis 8, dadurch **gekennzeichnet,** daß man
(a) eine Verbindung der Formel (II), worin R₁, R₂, R₅ und R₆ die in Anspruch 1 angegebenen Bedeutungen haben, mit dem Aminoester (III), bei dem R₃ und R₄ die in Anspruch 1 angegebenen Bedeutungen haben, umsetzt, 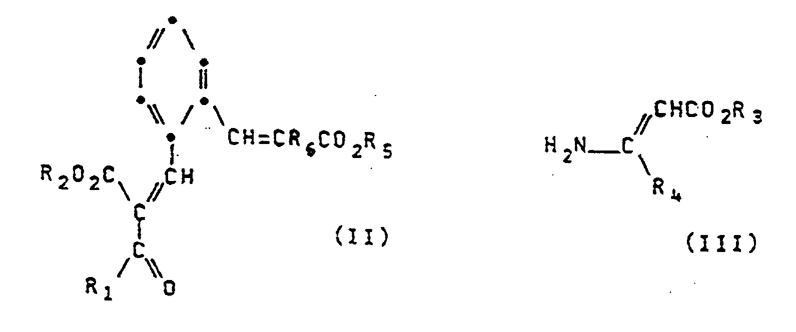
(b) und daß man den Aldehyd der Formel (IV) mit einem Aminoester der Formel (III) und dem Ketoester der Formel (V), wobei die Jeweiligen Gruppen R₆, R₅, R₄, R₃, R₂ und R₁ die in Anspruch 1 angegebenen Bedeutungen haben, umsetzt

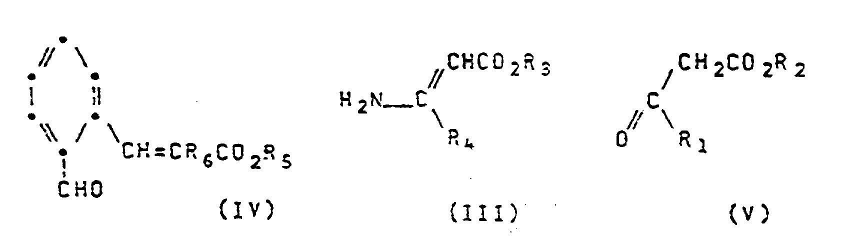

10. Pharmazeutische Präparate, dadurch **gekennzeichnet,** daß sie eine Verbindung nach einem der Ansprüche 1 bis 8 zusammen mit einem pharmazeutisch annehmbaren Träger oder Verdünnungsmittel enthalten.

11. Präparate nach Anspruch 10, dadurch **gekennzeichnet,** daß sie in einer Form vorliegen, die für die orale, sublinguale, transdermale, parenterale oder rektale Verabreichung oder für die Verabreichung durch Inhalation oder Insufflation geeignet ist.

12. Verbindung nach einem der Ansprüche 1 bis 8 oder ein physiologisch annehmbares Salz davon zur Verwendung als therapeutischer Wirkstoff.

## Patentansprüche (Patentansprüche für folgende(n) Vertragsstaat(en): AT, ES, GR)

1. Verfahren zur Herstellung von Verbindungen der allgemeinen Formel (I) 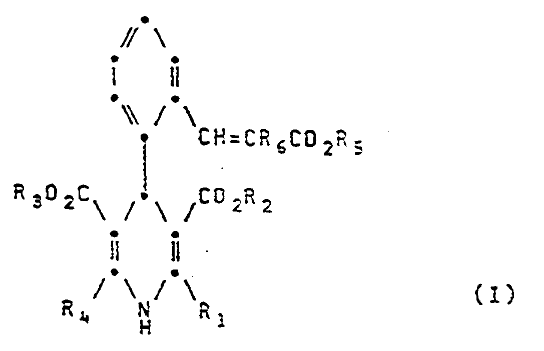 worin R₁ und R₄ unabhängig voneinander für eine C₁₋₄-Alkylgruppe stehen;
R₂ für eine Gruppe 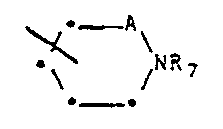 (worin A eine Bindung oder eine Methylengruppe ist und R₇ für Phenyl-C₁₋₄-Alkyl steht) steht; oder R₂ für eine Gruppe CH₂CH₂NR₈R₉ (worin R₈ für Wasserstoff oder C₁₋₄-Alkyl steht und R₉ für C₁₋₄-Alkyl, Phenyl-C₁₋₄-Alkyl oder Benzoyl-C₁₋₄-Alkyl steht) steht; oder R₂ für eine durch Nitril substituierte C₁₋₄-Alkylgruppe steht;
R₃ für eine geradkettige oder verzweigtkettige C₁₋₆-Alkyl- oder Alkoxyalkylgruppe steht;
R₅ für eine geradkettige oder verzweigtkettige C₂₋₉-Alkylgruppe steht; und
R₆ für ein Wasserstoff- oder Halogenatom oder eine C₁₋₃-Alkylgruppe steht,
und der physiologisch annehmbaren Salze davon, dadurch **gekennzeichnet,** daß man
(a) eine Verbindung der Formel (II), worin R₁, R₂, R₅ und R₆ die im Zusammenhang mit der Formel (I) angegebenen Bedeutungen haben, mit dem Aminoester (III), bei dem R₃ und R₄ die im Zusammenhang mit der Formel (I) angegebenen Bedeutungen haben, umsetzt, 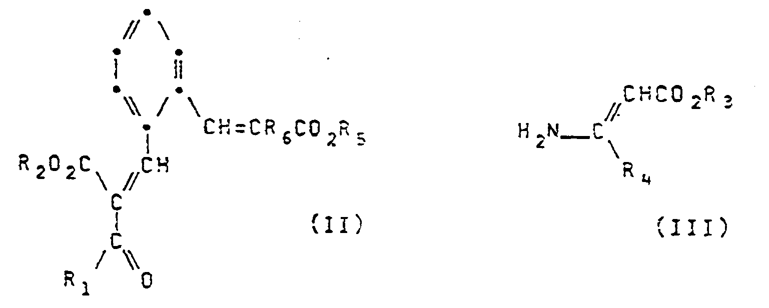
(b) und daß man den Aldehyd der Formel (IV) mit einem Aminoester der Formel (III) und dem Ketoester der Formel (V), wobei die jeweiligen Gruppen R₆, R₅, R₄, R₃, R₂ und R₁ die im Zusammenhang mit der Formel (I) angegebenen Bedeutungen haben, umsetzt

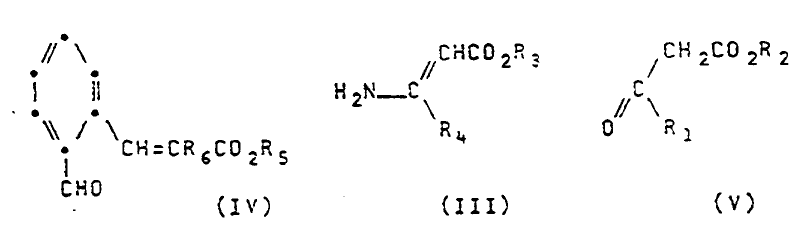

2. Verfahren nach Anspruch 1 zur Herstellung von Verbindungen, bei denen R₁ und R₄ für Methyl stehen.

3. Verfahren nach Anspruch 1 oder 2 zur Herstellung von Verbindungen, bei denen R₂ für N-Benzylpiperidino, CH₂CH₂CN oder CH₂CH₂NR₈R₉ (worin R₈ für Wasserstoff oder Methyl steht und R₉ für Methyl oder Benzyl steht) steht.

4. Verfahren nach einem der Ansprüche 1 bis 3 zur Herstellung von Verbindungen, bei denen R₃ für eine Methyl- oder Ethylgruppe steht.

5. Verfahren nach einem der Ansprüche 1 bis 4 zur Herstellung von Verbindungen, bei denen R₅ für eine tert.-Butylgruppe steht.

6. Verfahren nach einem der Ansprüche 1 bis 5 zur Herstellung von Verbindungen, bei denen R₆ für ein Wasserstoffatom steht.

7. Verfahren nach einem der Ansprüche 1 bis 6 zur Herstellung einer Verbindung, ausgewählt aus
2,6-Dimethyl-4-[2-(3-(1,1-dimethylethoxy)-3-oxo-1-propenyl)phenyl]-1,4-dihydro-3,5-pyridindicarbonsäureethyl-(1-benzyl-4-piperidinyl)ester;
2,6-Dimethyl-4-[2-(3-(1,1-dimethylethoxy)-3-oxo-1-propenyl)phenyl]-1,4-dihydro-3,5-pyridindicarbonsäureethyl-dimethylaminoethylester;
2,6-Dimethyl-4-[2-(3-(1,1-dimethylethoxy)-3-oxo-1-propenyl)phenyl]-1,4-dihydro-3,5-pyridindicarbonsäureethyl-2-cyanoethylester;
2,6-Dimethyl-4-[2-(3-(1,1-dimethylethoxy)-3-oxo-1-propenyl)phenyl]-1,4-dihydro-3,5-pyridindicarbonsäuremethyl-N-phenylmethyl-N-methylaminoethylester
und ihren physiologisch annehmbaren Salzen.

8. Verfahren nach den Ansprüchen 1 bis 7 zur Herstellung einer Verbindung, bei der das Wasserstoffatom und die Gruppe R₆ in der Gruppierung -CH=CR₆CO₂R₅ bezüglich einander in trans-Konfiguration vorliegen.

## Revendications (Revendications pour l'(les) Etat(s) contractant(s) suivant(s): BE, CH, DE, FR, GB, LI, LU, NL, SE)

1. Composés de la formule générale (I) 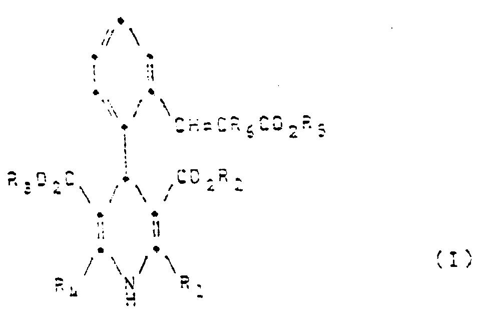 et de leurs sels physiologiquement acceptables, dans laquelle
R₁ et R₄ représentent indépendamment un groupe alkyle en C₁ à C₄;
R₂ représente un groupe 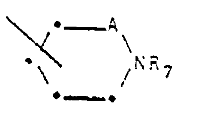 (dans lequel A est une liaison ou un groupe méthylène et R₇ est un phényl-alkyle en C₁ à C₄); ou R₂ représente un groupe CH₂CH₂NR₈R₉ (dans lequel R₈ est de l'hydrogène ou un alkyle en C₁ à C₄ et R₉ est un alkyle en C₁ à C₄, un phényl-alkyle en C₁ à C₄ ou un benzoyl-alkyle en C₁ à C₄); ou R₂ représente un groupe alkyle en C₁ à C₄ substitué par du nitrile;
R₃ représente un groupe alkyle ou alcoxyalkyle en C₁ à C₆, à chaîne droite ou ramifiée;
R₅ représente un groupe alkyle en C₂ à C₉, à chaîne droite ou ramifiée;
et
R₆ représente un atome d'hydrogène ou d'halogène ou bien un groupe alkyle en C₁ à C₃.

2. Composés tels que revendiqués dans la revendication 1, dans lesquels R₁ et R₄ représentent du méthyle.

3. Composés tels que revendiqués dans les revendications 1 ou 2, dans lesquels R₂ représente un radical N-benzylpipéridino, CH₂CH₂CN ou CH₂CH₂NR₈R₉ (dans lequel R₈ est de l'hydrogène ou du méthyle et R₉ est du méthyle ou du benzyle).

4. Composés tels que revendiqués dans l'une quelconque des revendications 1 à 3, dans lesquels R₃ représente un groupe méthyle ou éthyle.

5. Composés tels que revendiqués dans l'une quelconque des revendications 1 à 4, dans lesquels R₅ représente un groupe tert-butyle.

6. Composés tels que revendiqués dans l'une quelconque des revendications 1 à 5, dans lesquels R₆ représente un atome d'hydrogène.

7. Un composé choisi parmi les:
(1-benzyl-4-pipéridinyl) ester éthylique de l'acide 2,6-diméthyl-4-[2-(3-(1,1-diméthyléthoxy)-3-oxo-1-propényl)phényl]-1,4-dihydro-3,5-pyridine-dicarboxylique;
diméthylaminoéthyl ester éthylique de l'acide 2,6-diméthyl-4-[2-(3-(1,1-diméthyléthoxy)-3-oxo-1-propényl)phényl]-1,4-dihydro-3,5-pyridinedicarboxylique;
2-cyanoéthylester éthylique de l'acide 2,6-diméthyl-4-[2-(3-(1,1-diméthyléthoxy)-3-oxo-1-propényl)phényl]-1,4-dihydro-3,5-pyridinedicarboxylique;
N-phénylméthyl-N-méthylaminoéthyl ester méthylique de l'acide 2,6-diméthyl-4-[2-(3-(1,1-diméthyléthoxy)-3-oxo-1-propényl)phényl] -1,4-dihydro-3,5-pyridinedicarboxylique;
et leurs sels physiologiquement acceptables.

8. Un composé tel que revendiqué dans les revendications 1 à 7, dans lequel l'atome d'hydrogène et le groupe R₆ dans la partie -CH=CH₆CO₂R₅ sont trans l'un par rapport à l'autre.

9. Un procédé pour la préparation d'un composé de la formule générale (I) tel que défini dans l'une quelconque des revedications 1 à 8, procédé selon lequel:
a) on fait réagir un composé de formule la (II) dans laquelle R₁, R₂, R₅ et R₆ ont les significations spécifiées dans la revendication 1 avec l'aminoester (III) dans lequel R₃ et R₄ ont les significations spécifiées dans la revendication 1. 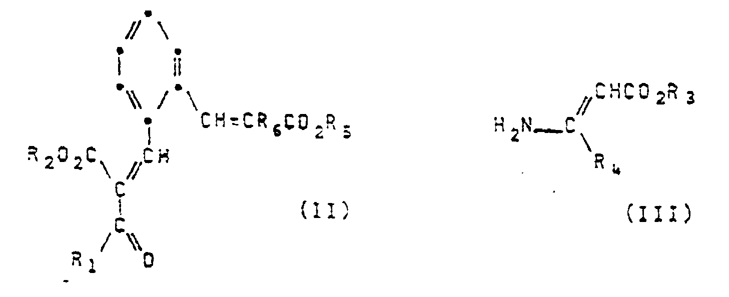
b) on fait réagir l'aldéhyde de la formule (IV) avec un aminoester de formule (III) et le cétoester de formule (V), dans laquelle les groupes respectifs R₆, R₅, R₄, R₃, R₂ et R₁ ont les significations spécifiées dans la revendication 1.

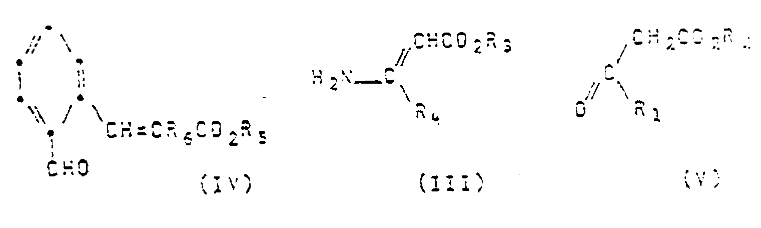

10. Composition pharmaceutique comportant un composé tel que revendiqué dans l'une quelconque des revendications 1 à 8 en association avec un support ou un diluant pharmaceutiquement acceptables.

11. Compositions telles que revendiquées dans la revendication 10 sous une forme appropriée pour l'administration orale, sublinguale, transdermique, parentérale, ou rectale ou bien pour l'administration par inhalation ou insuflation.

12. Un composé tel que revendiqué dans l'une quelconque des revendications 1 à 8 ou un de ses sels physiologiquement acceptables, destiné à être utilisé en tant qu'agent thérapeutique actif.

## Revendications (Revendications pour l'(les) Etat(s) contractant(s) suivant(s): AT, ES, GR)

1. Un procédé pour la préparation de composés de la formule générale (I) 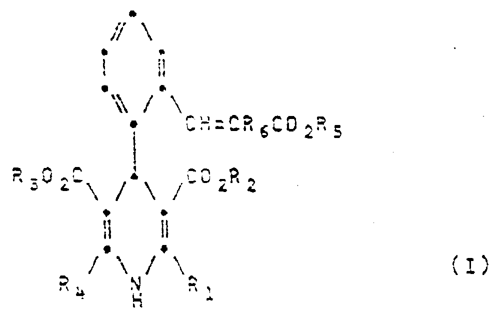 et de leurs sels physiologiquement acceptables, dans laquelle
R₁ et R₄ représentent indépendamment un groupe alkyle en C₁ à C₄;
R₂ représente un groupe 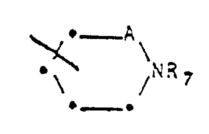 (dans lequel A est une liaison ou un groupe méthylène et R₇ est un phényl-alkyle en C₁ à C₄); ou R₂ représente un groupe CH₂CH₂NR₈R₉ (dans lequel R₈ est de l'hydrogène ou un alkyle en C₁ à C₄ et R₉ est un alkyle en C₁ à C₄, un phényl-alkyle en C₁ à C₄ ou un benzoyl-alkyle en C₁ à C₄); ou R₂ représente un groupe alkyle en C₁ à C₄ substitué par du nitrile;
R₃ représente un groupe alkyle ou alcoxyalkyle en C₁ à C₆, à chaîne droite ou ramifiée;
R₅ représente un groupe alkyle en C₂ à C₉, à chaîne droite ou ramifiée;
et
R₆ représente un atome d'hydrogène ou d'halogène ou bien un groupe alkyle en C₁ à C₃,
procédé selon lequel:
a) on fait réagir un composé de la formule (II) dans laquelle R₁, R₂, R₅ et R₆ ont les significations spécifiées dans la revendication 1 avec l'amino ester (III) dans lequel R₃ et R₄ ont les significations spécifiées dans la formule (I). 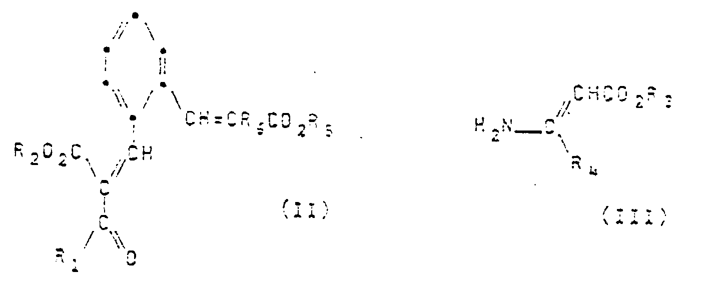
b) on fait réagir l'aldéhyde de la formule (IV) avec un aminoester de la formule (III) et le cétoester de formule (V), dans laquelle les groupes respectifs R₆, R₅, R₄, R₃, R₂ et R₁ ont les significations spécifiées dans la formule (I).

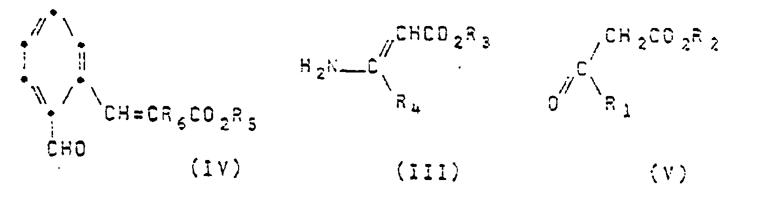

2. Un procédé tel que revendiqué dans la revendication 1, pour la préparation de composés dans lesquels R₁ et R₄ représentent du méthyle.

3. Un procédé tel que revendiqué dans la revendication 1 ou 2, pour la préparation de composés dans lesquels R₅ représente un radical N-benzylpipéridino, CH₂CH₂CN ou CH₂CH₂NR₈R₉ (dans lequel R₈ est de l'hydrogène ou du méthyle et R₉ est du méthyle ou du benzyle).

4. Un procédé tel que revendiqué dans l'une quelconque des revendications 1 à 3, pour la préparation de composés dans lesquels R₃ représente un groupe méthyle ou éthyle.

5. Un procédé tel que revendiqué dans l'une quelconque des revendications 1 à 4, pour la préparation de composés dans lesquels R₅ représente un groupe tert-butyle.

6. Un procédé tel que revendiqué dans l'une des revendications 1 à 5, pour la préparation de composés dans lesquels R₆ représente un atome d'hydrogène.

7. Un procédé tel que revendiqué dans l'une quelconque des revendications 1 à 6, pour la préparation d'un composé choisi parmi les:
(1-benzyl-4-piperidinyl) ester éthylique de l'acide 2,6-diméthyl-4-[2-(3-(1,1-diméthyléthoxy)3-oxo-1-propényl)phényl]-1,4-dihydro-3,5-pyridine-dicarboxylique;
diméthylaminoéthyl ester éthylique de l'acide 2,6-diméthyl-4-[2-(3-(1,1-diméthyléthoxy)-3-oxo-1-propényl)phényl]-1,4-dihydro-3,5-pyridinedicarboxylique;
2-cyanoéthylester éthylique de l'acide 2,6-diméthyl-4-[2-(3-(1,1-diméthyléthoxy)-3-oxo-1-propényl)phényl]-1,4-dihydro-3,5-pyridinedicarboxylique;
N-phénylméthyl-N-méthylaminoéthyl ester méthylique de l'acide 2,6-diméthyl-4-[2-(3-(1,1-diméthyléthoxy)-3-oxo-1-propényl)phényl] -1,4-dihydro-3,5-pyridinedicarboxylique;
et leurs sels physiologiquement acceptables.

8. Un procédé tel que revendiqué dans les revendications 1 à 7, pour la préparation d'un composé dans lequel l'atome d'hydrogène et le groupe R₆ dans la partie -CH=CR₆CO₂R₅ sont trans l'un par rapport à l'autre.
